# EUROPEAN PATENT SPECIFICATION

(11) **EP 4 162 877 B1**
(45) Date of publication and mention of the grant of the patent: **17.06.2026**
(21) Application number: 22200457.4
(22) Date of filing: 10.10.2022
(51) Int. Cl.: A61B 5/327, A61B 5/367, A61B 5/00

(54) **SUPPRESSING INTERFERENCE IN ELECTROCARDIOGRAM SIGNALS USING A TRAINED NEURAL NETWORK**
UNTERDRÜCKUNG VON INTERFERENZEN IN ELEKTROKARDIOGRAMMSIGNALEN UNTER VERWENDUNG EINES TRAINIERTEN NEURONALEN NETZWERKS
SUPPRESSION D'INTERFÉRENCE DANS DES SIGNAUX D'ÉLECTROCARDIOGRAMME À L'AIDE D'UN RÉSEAU NEURONAL ENTRAÎNÉ

(30) Priority: 11.10.2021 US 202163254323 P; 30.08.2022 US 202217898676
(43) Date of publication of application: 12.04.2023
(73) Proprietor: BIOSENSE WEBSTER (ISRAEL) LTD., 2066717 Yokneam (IL)
(72) Inventor: GOVARI, Assaf, 2066717 Yokneam (IL); ALTMANN, Andres Claudio, 2066717 Yokneam (IL); GLINER, Vadim, 2066717 Yokneam (IL)
(74) Representative: Carpmaels & Ransford LLP

(56) References cited:
- NIAN ZHANG ET AL: "Investigation of Adaptive Filtering for Noisy ECG Signals", ADAPTIVE AND LEARNING SYSTEMS, 2006 IEEE MOUNTAIN WORKSHOP ON, IEEE, PI, 1 July 2006 (2006-07-01), pages 30 - 35, XP031019173, ISBN: 978-1-4244-0166-6
- QIU YUE ET AL: "Elimination of power line interference from ECG signals using recurrent neural networks", 2017 39TH ANNUAL INTERNATIONAL CONFERENCE OF THE IEEE ENGINEERING IN MEDICINE AND BIOLOGY SOCIETY (EMBC), IEEE, 11 July 2017 (2017-07-11), pages 2296 - 2299, XP033152515, DOI: 10.1109/EMBC.2017.8037314
- KAROL ANTCZAK: "Deep Recurrent Neural Networks for ECG Signal Denoising", ARXIV.ORG, CORNELL UNIVERSITY LIBRARY, 201 OLIN LIBRARY CORNELL UNIVERSITY ITHACA, NY 14853, 30 July 2018 (2018-07-30), XP081120097
- VARSHNEY NEERAJ: "Combining electrocardiogram signal with Accelerometer signals for Human Activity Recognition using Convolution neural network", JOURNAL OF PHYSICS: CONFERENCE SERIES, vol. 1947, no. 1, 1 June 2021 (2021-06-01), GB, pages 012037, XP093192948, ISSN: 1742-6588, Retrieved from the Internet <URL:https://iopscience.iop.org/article/10.1088/1742-6596/1947/1/012037/pdf> DOI: 10.1088/1742-6596/1947/1/012037

## Description

### FIELD OF THE INVENTION

The present invention relates generally to medical devices, and particularly to methods and systems for suppressing interference in electrocardiogram signals.

### BACKGROUND OF THE INVENTION

Various techniques, such as application of a neural network, for improving the quality of signals acquired from an organ of a patient organ, are known in the art.

For example, U.S. Patent Application Publication 2020/0214597 describes sampling high-frequency (HF) QRS signals from a number of subjects (or derived values or features), and using e.g., deep learning-convolutional neural networks to find features or values which are (i) sufficiently similar for the same subject over all samples, yet (ii) sufficiently different among different subjects to allow identification. Also disclosed is finding signatures which are sufficiently stable over a particular period such that these signatures are within a deviation threshold, and then monitoring all subjects to be identified at least as often as the period used to establish the deviation threshold.

Indian Patent Application IN201841015767A describes extraction of foetal ECG from contaminated abdomen ECG, which is an integration of multi-rate signal processing, ANFIS algorithm, moving average filtering and wavelet denoising techniques. The invention is implemented in two phases, in the initial phase, the multi-rate processing technique is used where most of the maternal components are identified and removed. In the second phase, the remaining maternal components are identified through ANFIS algorithm with hybrid learning techniques and then removed. The extracted signal is further post processed to remove baseline wander noise and other noise components to obtain clean foetal ECG.

U.S. Patent Application Publication 2020/0260980 describes a self-learning dynamic electrocardiography analysis method employing artificial intelligence. The method comprises: pre-processing data, performing cardiac activity feature detection, interference signal detection and cardiac activity classification on the basis of a deep learning method, performing signal quality evaluation and lead combination, examining cardiac activity, performing analytic computations on an electrocardiogram event and parameters, and then automatically outputting report data. The method achieves an automatic analysis method for a quick and comprehensive dynamic electrocardiography process, and recording of modification information of an automatic analysis result, while also collecting and feeding back modification data to a deep learning model for continuous training, thereby continuously improving and enhancing the accuracy of the automatic analysis method. Also disclosed is a self-learning dynamic electrocardiography analysis device employing artificial intelligence.

Nian Zang et al ("Investigation of Adaptive Filtering for Nois ECG signals", Adaptive Deep Learning Systems, 2006, IEEE Mountain Workshop ON, IEEE, PI, 1 July2006, pp.30-35 ISBN:978-1-4244-0166-6) discusses development of an adaptive filter to remove the a contaminating signal from an ECG signal and compares fault-tolerant and non-fault-tolerant adaptive filters.

Karol Antczak ("Deep Recurrent Neural Networks for ECG Signal Denoising", arxiv.org, Cornell University Library, 201 Olin Library Cornell University Ithaca, NY14853, 30th July 2018) discusses an approach to denoising electrocardiograph signals with deep recurrent neural networks. Such an approach utilizes a transfer learning technique by pretraining the network using synthetic data, generated by a dynamic ECG model, and fine-tuning it with real data.

Qiu Yue et al ("Elimination of power line interference from ECG signals using recurrent neural networks", 2017 39th Annual International Conference of the IEEE Engineering in Medicine and Biology Society (EMBC), IEEE, 11 July 2017, pp.2296-2299, DOI: 10.1109/EMBC.2017.8037314) discusses method for the elimination of 50-Hz power line interference (PLI) in electrocardiogram (ECG) signals, using recurrent neural networks (RNN). The method is to preliminarily train an RNN-based model to obtain the signal pattern of PLI. The ECG signals are then filtered by subtracting the PLI signals extracted by the trained model.

### SUMMARY OF THE INVENTION

An embodiment of the present invention that is described herein provides a method as defined in claim 4.

The present invention will be more fully understood from the following detailed description, taken together with the drawings in which:

### BRIEF DESCRIPTION OF THE DRAWINGS

Fig. 1 is a schematic, pictorial illustration of a catheter-based tracking and ablation system, in accordance with an embodiment of the present invention;
Fig. 2A is a schematic, block diagram illustrating training of a neural network (NN) for suppressing interference in distorted ECG signals, in accordance with an embodiment of the present invention;
Fig. 2B is a schematic, block diagram illustrating applying the trained NN for producing ECG signal in which interference is suppressed relative to a distorted ECG signal received from a heart of a patient, in accordance with an embodiment of the present invention; and
Fig. 3 is a flow charts that schematically illustrates a method for suppressing interference in distorted real-time ECG signals received from the heart of the patient, in accordance with an embodiment of the present invention.

### DETAILED DESCRIPTION OF EMBODIMENTS

### OVERVIEW

During a medical procedure, such as procedure involving electrophysiological (EP) mapping, electrocardiogram (ECG) signals acquired from a patient heart may be distorted by interference from other signals received from one or more sources external to the patient heart. One example source of such signals may comprise a spectral line of the power grid having a frequency of about 50 Hz or 60 Hz, and one or more harmonics of at least one of these spectral lines.

In principle, it is possible to use various types of algorithms or other techniques in order to suppress at least part of the interference and produce an undistorted ECG signal. However, the interference suppression in such techniques may require a long time and producing the undistorted ECG signal must be carried out in real-time, e.g., when acquiring the ECG signal. Moreover, it is important to present the undistorted ECG signal to a physician in real-time, e.g., while performing the medical procedure.

Embodiments of the present invention that are described hereinbelow provide techniques for improving the quality of distorted ECG signals acquired from patient heart, wherein the quality improvement is carried out in real time, e.g., within less than one second from receiving the distorted ECG signal(s). The distortion may be caused by a spectral line of the power grid signals and respective harmonics thereof, as described above.

The system for improving the quality of ECG signals, by suppressing the spectral line and harmonics of the power grid signals, comprises an interface and a processor.

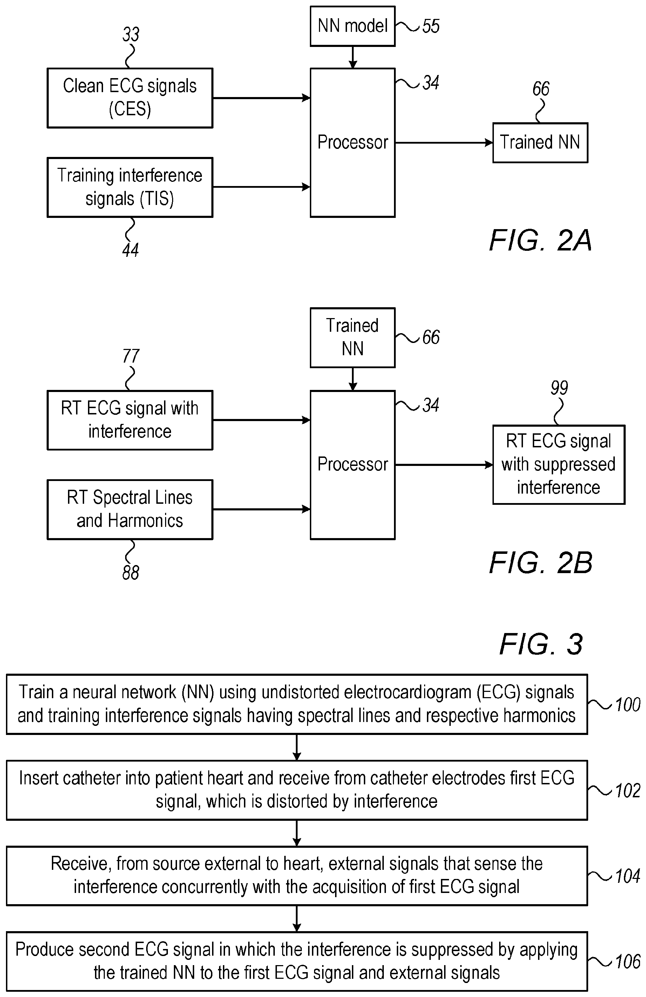

In some embodiments, the processor is configured to receive a model of a neural network (NN), such as an autoencoder artificial NN having at least five layers, and typically about ten layers, examples of NN models are described in Fig. 2A below.

The processor is configured to train the NN using one or more training ECG signals that are not distorted by the interference of the aforementioned power signals. The training ECG signals may be prepared in advanced, for example, by suppressing distortions in natural ECG signals, or by producing synthetic ECG signals having features of interest, e.g., for a specific heart disease.

The processor is configured to train the NN using, in addition to the training ECG signals, one or more training interference signals each having one or more respective spectral lines and one or more respective harmonics. For example, the spectral lines and harmonics of the training interference signals may have one or more frequencies, which are typical to power signals of the electrical grid e.g., about 50 Hz or 60 Hz, used in Europe-Asia or in North America, respectively. After concluding the training, the processor has a trained NN that may be implemented in software or in hardware or in a suitable combination thereof.

After training the NN and during an EP mapping, the interface is configured to receive an ECG signal, which is acquired in the patient heart and is also referred to herein as a first ECG signal. In some cases, the first ECG signal is distorted by interference caused by the spectral line and harmonics of the power grid signals.

In some embodiments, the interface is also configured to receive, from one or more sources external to the heart, one or more external signals that sense the interference concurrently with the acquisition of the first ECG signal (e.g., during the EP mapping).

The processor is configured to apply the trained NN to the first ECG signal and to the one or more external signals, so as to produce a second ECG signal, in which the interference is suppressed relative to the first ECG signal. Note that by applying the trained NN to the first ECG signal, the processor may produce the second ECG signal in real-time (e.g., within less than one second after receiving the first ECG signal). Moreover, the processor is configured to immediately present the second ECG signal to the physician while performing the EP mapping (instead of or in addition to presenting the first ECG signal).

The disclosed techniques improve the accuracy of electro-anatomical (EA) mapping, and therefore, improve the efficiency and quality of medical procedures that are based on the EA mapping.

### SYSTEM DESCRIPTION

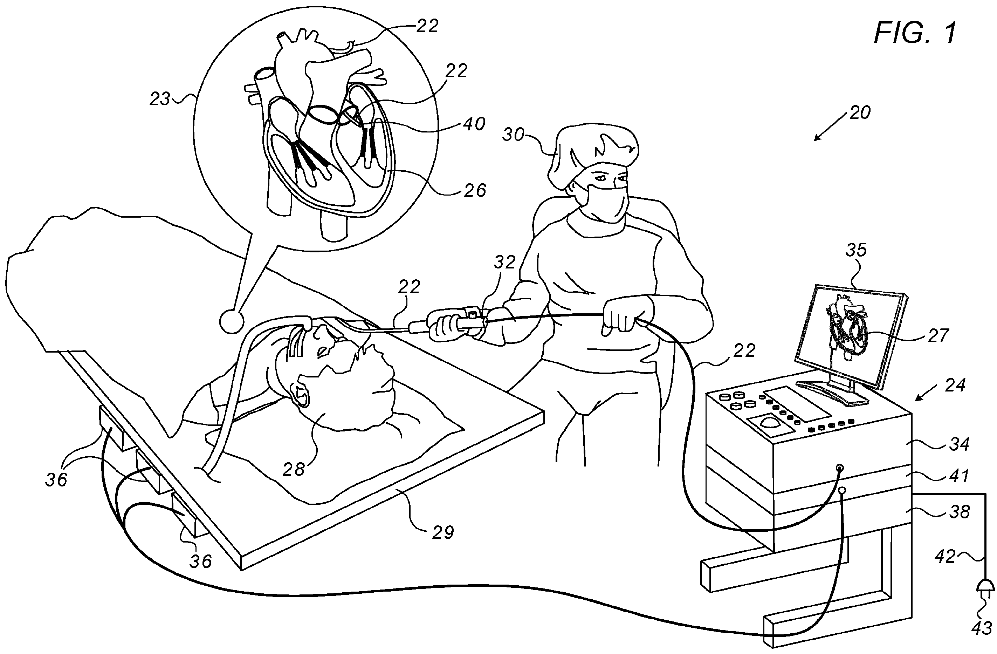

Fig. 1 is a schematic, pictorial illustration of a catheter-based tracking and ablation system 20, in accordance with an embodiment of the present invention.

In some embodiments, system 20 comprises a catheter 22, in the present example a cardiac catheter, and a control console 24. In the embodiment described herein, catheter 22 may be used for any suitable therapeutic and/or diagnostic purposes, such as sensing electro-anatomical signals in a heart 26.

In some embodiments, console 24 comprises a processor 34, typically a general-purpose computer, with suitable front end and interface circuits for receiving signals via catheter 22 and for controlling the other components of system 20 described herein. Console 24 further comprises a user display 35, which is configured to receive from processor 34 a map 27 of heart 26, and to display map 27.

In some embodiments, map 27 may comprise any suitable type of three-dimensional (3D) anatomical map produced using any suitable technique. For example, the anatomical map may be produced using an anatomical image produced by using a suitable medical imaging system, or using a technique denoted fast anatomical mapping (FAM), which is available in the CARTO^{™} system, provided by Biosense Webster Inc. (Irvine, Calif.), or using any other suitable technique, or using any suitable combination of the above.

Reference is now made to an inset 23. In some embodiments, prior to performing an ablation procedure, a physician 30 inserts catheter 22 through the vasculature system of a patient 28 lying on a table 29, so as to perform electro-anatomical (EA) mapping of tissue in question of heart 26. During the EA mapping, physician 30 controls catheter 22 to sense one or more electrocardiogram (ECG) signal(s) acquired in tissue of heart 26, as will be described herein.

In some embodiments, catheter 22 comprises a distal-end assembly 40 having multiple sensing electrodes (not shown). For example, distal-end assembly 40 may comprise: (i) a basket catheter having multiple splines, each spline having multiple sensing electrodes, (ii) a balloon catheter having multiple sensing electrodes disposed on the surface of the balloon, or (iii) a focal catheter (shown in the example of Fig. 1) having multiple sensing electrodes.

In some embodiments, in response to sensing in tissue of heart 26 electrophysiological (EP) signals such as ECG signals, each sensing electrode is configured to produce one or more signals indicative of the sensed ECG signals.

In some embodiments, the proximal end of catheter 22 is connected, *inter alia,* to interface circuits, referred to herein as interface 38, or to the interface circuits of processor 34, so as to transfer the ECG signals to processor 34 for performing the EA mapping. In some embodiments, during the EA mapping, the signals produced by the sensing electrodes of distal-end assembly 40 may comprise thousands of data points, e.g., about 50,000 data points or even more, which may be stored in a memory (not shown) of console 24. Based on the data points, processor 34 is configured to present on map 27, wave vectors, also referred to herein as vectors, which are indicative of electrical signals propagating over the surface of heart 26.

In the context of the present disclosure and in the claims, the terms "about" or "approximately" for any numerical values or ranges indicate a suitable dimensional tolerance that allows the part or collection of components to function for its intended purpose as described herein.

In some cases, one or more of the acquired ECG signals may be distorted by interference that may undesirably be received from one or more sources external to heart 26. For example, an alternating current (AC) power signals of an electrical power grid 43 has a typical frequency of about 60 Hz (used in the grid of North America and Japan) or 50 Hz (used in the grid of Europe, most Asian countries, and in other continents) and one or more respective harmonics, may undesirably interfere with the acquired ECG signals. The power signals are referred to herein as "external signals" because they are produced externally to heart 26, and are conducted into system 20 via a cable 42, e.g., into interface 38. Such external signals may be sensed concurrently with the acquisition of the ECG signals sensed by the electrodes of distal-end assembly 40. For example, the external signals may be transferred to processor 34 via cable 42 and catheter 22 into interface 38, and therefore, may distort the ECG signals. Techniques for suppressing the interference are disclosed in detail in Figs. 2A, 2B and 3 below.

In other embodiments, catheter 22 may comprise one or more ablation electrodes (not shown) coupled to distal-end assembly 40. The ablation electrodes are configured to ablate tissue at a target location of heart 26, which is determined based on the analysis of the EA mapping of the tissue in question of heart 26. After determining the ablation plan, physician 30 navigates distal-end assembly 40 in close proximity to the target location in heart 26 e.g., using a manipulator 32 for manipulating catheter 22. Subsequently, physician 30 places one or more of the ablation electrodes in contact with the target tissue, and applies, to the tissue, one or more ablation signals. Additionally, or alternatively, physician 30 may use any different sort of suitable catheter for ablating tissue of heart 26 so as to carry out the aforementioned ablation plan.

In some embodiments, the position of distal-end assembly 40 in the heart cavity is measured using a position sensor (not shown) of a magnetic position tracking system, which is coupled to distal-end assembly 40. In the present example, console 24 comprises a driver circuit 41, which is configured to drive magnetic field generators 36 placed at known positions external to patient 28 lying on table 29, e.g., below the patient's torso. The position sensor is coupled to the distal end, and is configured to generate position signals in response to sensed external magnetic fields from field generators 36. The position signals are indicative of the position the distal end of catheter 22 in the coordinate system of the position tracking system.

This method of position sensing is implemented in various medical applications, for example, in the CARTO^{™} system, produced by Biosense Webster Inc. (Irvine, Calif.) and is described in detail in U.S. Patents 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1.

In some embodiments, the coordinate system of the position tracking system is registered with the coordinate systems of system 20 and map 27, so that processor 34 is configured to display the position of distal-end assembly 40, over the anatomical or EA map (e.g., map 27).

In some embodiments, processor 34, typically comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

This particular configuration of system 20 is shown by way of example, in order to illustrate certain problems that are addressed by embodiments of the present invention and to demonstrate the application of these embodiments in enhancing the performance of such a system. Embodiments of the present invention, however, are by no means limited to this specific sort of example system, and the principles described herein may similarly be applied to other sorts of medical systems.

### TRAINING NEURAL NETWORK FOR SUPPRESSING INTERFERENCE IN ECG SIGNALS

Fig. 2A is a schematic, block diagram illustrating training of a neural network (NN), also referred to herein as a NN model 55, for suppressing interference in distorted ECG signals, in accordance with an embodiment of the present invention.

In the example of Figs. 2A and 2B, NN model 55 is implemented in software, which is processed in processor 34 or in any other suitable sort of a processing device. In other embodiments, NN model 55 may be at least partially implemented in hardware, for example, as a module of processor 34 or in another electronic device (not shown) configured to exchange signals with processor 34.

The NN model 55 may comprise any suitable type of NN, such as but not limited an autoencoder artificial NN, e.g., regularized autoencoders, concrete autoencoder, variational autoencoder (VAE), or other suitable types of NNs used for suppressing interference (e.g., denoising). For example, NN model 55 may have about ten (10) layers (or any other suitable number of layers, e.g., at least five layers) and may be based on the architecture of AlexNet NN supplied by Alex Krizhevsky (Toronto, Canada), or on the TensorFlow open-source machine learning platform supplied by Google AI, a subsidiary of Google (Mountain View, California, US), or any other suitable type of NN architecture.

The processor 34 is configured to receive, e.g., via interface 38, one or more training ECG signals that are not distorted by various types of interference, such as the interference described in Fig. 1 above. In the example of Fig. 2A, the training ECG signals are also referred to herein and shown as clean ECG signals (CES) 33.

The CES 33 may comprise undistorted ECG signals received from the aforementioned CARTO^{™} system having the data arranged in 2.5 second blocks. For example, the signal from the sensing electrodes of distal-end assembly 40 may be sampled at a frequency of about 1 kHz, thus, each clean ECG signal 33 comprises about 2,500 points used for training NN model 55. Additionally, or alternatively, one or more of CES 33 may comprise any other suitable clean ECG signals received from other sources, or synthetic CES produced by using any suitable models.

During the training of NN model 55, processor 34 is configured to receive, e.g., via interface 38, one or more training interference signals (TIS) 44, each training interference signal 44 has one or more spectral lines (e.g., 50 Hz or 60 Hz received from electrical power grid 43, as described in Fig. 1 above) and one or more respective harmonics of each spectral line. Note that TIS 44 may comprise one or more power signals received samples from electrical power grid 43, or other signals (sampled and/or produced synthetically) having other suitable spectral lines and harmonics thereof.

After training NN model 55 using sufficient examples (e.g., about 100000 samples or any other suitable number of samples) of CES 33 and TIS 44, processor 34 is configured to output a trained NN 66.

Note that in the present example, trained NN 66 is implemented in software. In other embodiments, e.g., when at least part of NN model 55 is implemented in hardware, then at least part of trained NN 66 is also implemented in hardware.

### APPLYING NEURAL NETWORK FOR SUPPRESSING INTERFERENCE IN ECG SIGNALS

Fig. 2B is a schematic, block diagram illustrating the application of trained NN 66 for suppressing interference in a distorted ECG signal sensed in heart 26, in accordance with an embodiment of the present invention.

In some embodiments, during the EA mapping processor 34 is configured to receive from the sensing electrodes of distal-end assembly 40 placed in contact with heart 26 and via interface 38 (e.g., via interface 38), a real-time ECG signal (RTES) 77 that is typically distorted by interference as described in Figs. 1 and 2A above.

In some embodiments, processor 34 is configured to receive, from one or more sources external to heart 26 (e.g., electrical power grid 43 received via cable 42 and interface 38), one or more real-time external signals (RTEX) 88, also referred to herein as real-time spectral lines and harmonics. In the context of the present disclosure, the term "real-time" refers to a time interval of the EA mapping procedure, e.g., when the ECG signals are sensed in heart 26 by the sensing electrodes of distal-end assembly 40.

In some embodiments, processor 34 is configured to apply trained NN 66 for producing, during the EA mapping procedure, a real-time clean ECG signal (RTCES) 99, which is an ECG signal in which the interference is suppressed relative to RTES 77, which is the distorted ECG signal received from heart 26 of patient 28. In other words, the ECG signal received from heart 26 is distorted by "line noise" interference, e.g., interference of power signals received from electrical power grid 43. Trained neural network 66 is applied to the distorted ECG signal for removing (e.g., subtracting) the line noise, and for producing a "cleaner" ECG signal, such as RTCES 99, in which at least the "line noise" interference is removed.

In some embodiments, when applying trained NN 66 to the acquired ECG signals, the process of "cleaning" the signal acquired from heart 26 (e.g., RTES 77) is carried out immediately (e.g., within less than one second), so that processor 34 may display the corresponding "clean" ECG signal, e.g., RTCES 99, to physician 30. Note that the properties of the signal acquired from heart 26 (e.g., RTES 77) are retained in the corresponding "clean" ECG signal (e.g., RTCES 99), and only the interference causing the distortion is being removed.

Fig. 3 is a flow charts that schematically illustrates a method for suppressing interference in distorted real-time ECG signals 77 received from heart 26 of patient 28, in accordance with an embodiment of the present invention.

The method begins at a neural network training step 100, with training NN model 55, e.g., in processor 34, using undistorted ECG signals, such as CES 33, and training interference signals having spectral lines and respective harmonics, such as TIS 44. Step 100 is concluded with obtaining trained NN 66, as described in Fig. 2A above.

At an ECG sensing step 102, distal-end assembly 40 of catheter 22 is inserted into heart 26 for performing EA mapping, and the sensing electrodes of distal-end assembly 40 are used for acquiring a first ECG signal that is distorted by interference, such as RTES 77, and is received by processor 34, as described in Fig. 2B above.

At an external signal receiving step 104, processor 34 receives from one or more sources external to heart 26, external signals (e.g., RTEX 88) that comprise the interference sensed concurrently with the acquisition of the first ECG signal (e.g., RTES 77). In the present example, RTEX 88 is based on signals received from electrical power grid 43 received via cable 42 and interface 38, as described in Figs. 1 and 2B above.

At a clean ECG signal production step 106 that concludes the method, processor 34 applies trained NN 66 to the first ECG signal (e.g., RTES 77) and to the external signal (e.g., RTEX 88) for producing a second ECG signal (e.g., RTCES 99) in which the interference in RTES 77 is suppressed, as described in Fig. 2B above.

Although the embodiments described herein mainly address improving the quality of ECG signals sensed in a patient heart, the methods and systems described herein can also be used in other applications, such as in electroencephalogram (EEG) procedures.

The scope of invention is as defined in the appended independent claims, preferred embodiments are defined in the dependent claims.

## Claims

1. A method, comprising:
receiving a first electrocardiogram (ECG) signal (77), which is acquired in a heart of a patient and is distorted by interference;
receiving, from one or more sources external to the heart, one or more external signals (88) that sense the interference concurrently with acquisition of the first ECG signal; and
producing a second ECG signal (99), in which the interference is suppressed relative to the first ECG signal (33), by applying a trained Neural Network (NN, 66) to the first ECG signal (77) and to the one or more external signals (88) ;
wherein the interference (88) comprises one or more spectral lines and one or more harmonics of the one or more spectral lines;
the method further comprising training the NN using (i) one or more training ECG signals (33) that are not distorted by the interference, and (ii) one or more training interference signals (44) each having one or more respective spectral lines and one or more respective harmonics.

2. The method according to claim 1, wherein training the NN comprises training an autoencoder artificial NN having at least five layers.

3. The method according to claim 1, wherein the at least one of the spectral lines comprises a frequency of an alternating current (AC) of a power signal.

4. A system, comprising:
an interface, which is configured to receive: (i) a first electrocardiogram (ECG) signal (77), which is acquired in a heart of a patient and is distorted by interference, and (ii) one or more external signals (88) that are received from one or more sources external to the heart, and sense the interference concurrently with acquisition of the first ECG signal; and
a processor (34), which is configured to produce a second ECG signal (99), in which the interference is suppressed relative to the first ECG signal (77), by applying a trained Neural Network (NN, 66) to the first ECG signal (77) and to the one or more external signals (88);
wherein the interference comprises one or more spectral lines and one or more harmonics of the one or more spectral lines; and
the processor is further configured to train the NN using (i) one or more training ECG signals (33) that are not distorted by the interference, and (ii) one or more training interference signals (44) each having one or more respective spectral lines and one or more respective harmonics.

5. The system according to claim 4, wherein the processor is configured to train an autoencoder artificial NN having at least five layers.

6. The system according to claim 4, wherein the at least one of the spectral lines comprises a frequency of an alternating current (AC) of a power signal.

## Patentansprüche

1. Verfahren, umfassend:
Empfangen eines ersten Elektrokardiogrammsignals (EKG-Signal) (77), das in einem Herzen eines Patienten erfasst wird und durch Interferenz verzerrt ist;
Empfangen von einer oder mehreren externen Quellen außerhalb des Herzens eines oder mehrerer externer Signale (88), die die Interferenz gleichzeitig mit der Erfassung des ersten EKG-Signals erfassen; und
Erzeugen eines zweiten EKG-Signals (99), in dem die Interferenz relativ zu dem ersten EKG-Signal (33) unterdrückt ist, durch Anwenden eines trainierten neuronalen Netzwerks (NN, 66) auf das erste EKG-Signal (77) und auf das eine oder die mehreren externen Signale (88);
wobei die Interferenz (88) eine oder mehrere Spektrallinien und eine oder mehrere Harmonische der einen oder mehreren Spektrallinien umfasst;
das Verfahren ferner umfassend das Trainieren des NN unter Verwendung von (i) einem oder mehreren Trainings-EKG-Signalen (33), die nicht durch die Interferenz verzerrt sind, und (ii) einem oder mehreren Trainingsinterferenzsignalen (44), die jeweils eine oder mehrere entsprechende Spektrallinien und eine oder mehrere entsprechende Harmonische aufweisen.

2. Verfahren nach Anspruch 1, wobei das Trainieren des NN das Trainieren eines Autocodierers mit künstlichem NN mit mindestens fünf Schichten umfasst.

3. Verfahren nach Anspruch 1, wobei die mindestens eine der Spektrallinien eine Frequenz eines Wechselstroms (AC) eines Leistungssignals umfasst.

4. System, umfassend:
eine Schnittstelle, die konfiguriert ist für den Empfang von: (i) einem ersten Elektrokardiogrammsignal (EKG-Signal) (77), das in einem Herzen eines Patienten erfasst wird und durch Interferenz verzerrt ist, und (ii) ein oder mehrere externe Signale (88), die von einer oder mehreren Quellen außerhalb des Herzens empfangen werden und die Interferenz gleichzeitig mit der Erfassung des ersten EKG-Signals erfassen; und
einem Prozessor (34), der konfiguriert ist, um ein zweites EKG-Signal (99) zu erzeugen, in dem die Interferenz relativ zu dem ersten EKG-Signal (77) unterdrückt wird, durch Anwenden eines trainierten neuronalen Netzwerks (NN, 66) auf das erste EKG-Signal (77) und auf das eine oder die mehreren externen Signale (88);
wobei die Interferenz eine oder mehrere Spektrallinien und eine oder mehrere Harmonische der einen oder mehreren Spektrallinien umfasst; und
der Prozessor ferner konfiguriert ist, um das NN unter Verwendung von (i) einem oder mehreren Trainings-EKG-Signalen (33), die nicht durch die Interferenz verzerrt sind, und (ii) einem oder mehreren Trainingsinterferenzsignalen (44), die jeweils eine oder mehrere entsprechende Spektrallinien und eine oder mehrere entsprechende Harmonische aufweisen, zu trainieren.

5. System nach Anspruch 4, wobei der Prozessor konfiguriert ist, um einen Autocodierer mit künstlichem NN mit mindestens fünf Schichten zu trainieren.

6. System nach Anspruch 4, wobei die mindestens eine der Spektrallinien eine Frequenz eines Wechselstroms (AC) eines Leistungssignals umfasst.

## Revendications

1. Procédé, comprenant :
la réception d'un premier signal d'électrocardiogramme (ECG) (77), qui est acquis dans le cœur d'un patient et est déformé par l'interférence ;
la réception, d'une ou plusieurs sources externes au cœur, d'un ou plusieurs signaux externes (88) qui détectent l'interférence en même temps que l'acquisition du premier signal ECG ; et
la production d'un second signal ECG (99), dans lequel l'interférence est supprimée par rapport au premier signal ECG (33), en appliquant un réseau neuronal entraîné (NN, 66) au premier signal ECG (77) et au ou aux signaux externes (88) ;
dans lequel l'interférence (88) comprend une ou plusieurs raies spectrales et une ou plusieurs harmoniques de la ou des raies spectrales ;
le procédé comprenant en outre l'entraînement du NN à l'aide (i) d'un ou plusieurs signaux ECG d'entraînement (33) qui ne sont pas déformés par l'interférence, et (ii) d'un ou plusieurs signaux d'interférence d'entraînement (44) ayant chacun une ou plusieurs raies spectrales respectives et une ou plusieurs harmoniques respectives.

2. Procédé selon la revendication 1, dans lequel l'entraînement du NN comprend l'entraînement d'un NN artificiel d'autoencodeur ayant au moins cinq couches.

3. Procédé selon la revendication 1, dans lequel l'au moins une des raies spectrales comprend une fréquence d'un courant alternatif (CA) d'un signal d'alimentation.

4. Système, comprenant :
une interface, qui est configurée pour recevoir : (i) un premier signal d'électrocardiogramme (ECG) (77), qui est acquis dans le cœur d'un patient et est déformé par l'interférence, et (ii) un ou plusieurs signaux externes (88) qui sont reçus d'une ou de plusieurs sources externes au cœur, et qui détectent l'interférence en même temps que l'acquisition du premier signal ECG ; et
un processeur (34), qui est configuré pour produire un second signal ECG (99), dans lequel l'interférence est supprimée par rapport au premier signal ECG (77), en appliquant un réseau neuronal entraîné (NN, 66) au premier signal ECG (77) et au ou aux signaux externes (88) ;
dans lequel l'interférence comprend une ou plusieurs raies spectrales et une ou plusieurs harmoniques de la ou des raies spectrales ; et
le processeur est en outre configuré pour entraîner le NN à l'aide (i) d'un ou plusieurs signaux ECG d'entraînement (33) qui ne sont pas déformés par l'interférence, et (ii) d'un ou plusieurs signaux d'interférence d'entraînement (44) ayant chacun une ou plusieurs raies spectrales respectives et une ou plusieurs harmoniques respectives.

5. Système selon la revendication 4, dans lequel le processeur est configuré pour entraîner un autoencodeur artificiel, NN, ayant au moins cinq couches.

6. Système selon la revendication 4, dans lequel l'au moins une des lignes spectrales comprend une fréquence d'un courant alternatif (CA) d'un signal de puissance.
